# EUROPÄISCHE PATENTSCHRIFT

(11) **EP 1 790 292 B1**
(45) Veröffentlichungstag und Bekanntmachung des Hinweises auf die Patenterteilung: **16.01.2008**
(21) Anmeldenummer: 05025706.2
(22) Anmeldetag: 24.11.2005
(51) Int. Cl.: A61B 17/00, B25B 23/00, B25G 1/00

(54) **Chirurgische Kupplungsvorrichtung**
Surgical coupling device
Dispositif d'accouplement chirurgical

(43) Veröffentlichungstag der Anmeldung: 30.05.2007
(73) Patentinhaber: EFS - Eberle-Feinwerktechnische Systeme GmbH & Co. KG, 75449 Wurmberg (DE)
(72) Erfinder: Eberle, Frank, 75449 Wurmberg (DE)
(74) Vertreter: Weber, Joachim

(56) Entgegenhaltungen:
- US-A- 2 682 414

## Beschreibung

Die Erfindung bezieht sich auf eine chirurgische Kupplungsvorrichtung zur lösbaren Verbindung eines Handstücks mit einem chirurgischen Instrument.

Chirurgische Instrumente, welche in ein Handstück eingesetzt werden, sind in unterschiedlichsten Ausgestaltungsformen bekannt. Die Handstücke sind üblicherweise mit einem Antrieb verbunden, so dass Werkzeuge oder Teilbereiche der chirurgischen Instrumente antreibbar, beispielsweise in Drehung versetzbar sind. Derartige chirurgische Instrumente können beispielsweise zahnärztliche Bohrer oder Fräser sein, sie können auch als Shaver-Instrumente für die Arthroskopie ausgebildet sein.

Aus dem Stand der Technik ist es bekannt (siehe beispielsweise EP 1 006 898 B1), das Handstück mit einer zentrischen Bohrung oder Ausnehmung zu versehen, in welche ein Kupplungsbereich oder Ansatzbereich des chirurgischen Instruments eingesteckt wird. Dabei gelangen entsprechende Antriebskupplungen des Handstücks in Eingriff mit Kupplungsteilen des chirurgischen Instruments, so dass ein Drehantrieb realisiert wird. Das Einstecken und Verrasten des chirurgischen Instruments in dem Handstück erfolgt dadurch, dass das Handstück mit einer Verrastungsausnehmung versehen ist, mit welcher ein Rastelement des chirurgischen Instruments lösbar in Eingriff bringbar ist. Die Verrastungsausnehmung kann beispielsweise in Form einer Ringnut ausgebildet sein, es ist auch möglich, diese nur singulär vorzusehen. Das Rastelement des chirurgischen Instruments kann beispielsweise in Form eines Rasthakens, einer Rastklinke oder Ähnlichem ausgebildet sein. Beim Einstecken des chirurgischen Instruments in das Handstück kann somit eine Verrastung oder Kupplung erfolgen, die ein unbeabsichtigtes Ablösen des chirurgischen Instruments von dem Handstück verhindert. Um das chirurgische Instrument von dem Handstück zu entnehmen, ist beispielsweise eine Betätigungseinrichtung zu bedienen.

Die aus dem Stand der Technik bekannten Vorrichtungen sind vielfach kompliziert aufgebaut und entsprechend kostenintensiv und aufwendig in der Herstellung und unter operativen Einsatzbedingungen vielfach schlecht betätigbar.

Die US 2 682 414 A zeigt einen längeneinstellbaren Schraubendreher mit einem Griff sowie einem Werkzeugeinsatz, welcher in einer zentrischen Längsausnehmung des Griffes verschiebbar gelagert ist. Der Griff trägt eine Wippe, deren freies Ende mit einer Klinke versehen ist, die in eine von mehreren Ausnehmungen des Werkzeugs einrastbar ist. Die Wippe wird durch einen an ihrem anderen Ende angeordneten, federvorgespannten Betätigungsknopf verschwenkt, um das Werkzeug zu dessen Verschiebung freizugeben.

Der Erfindung liegt die Aufgabe zugrunde, eine chirurgische Kupplungsvorrichtung der eingangs genannten Art zu schaffen, welche bei einfachem Aufbau und einfacher, kostengünstiger Herstellbarkeit sicher und einfach bedienbar ist.

Erfindungsgemäß wird die Aufgabe durch die Merkmalskombination des Hauptanspruchs gelöst, die Unteransprüche zeigen weitere vorteilhafte Ausgestaltungen der Erfindung.

Erfindungsgemäß ist somit vorgesehen, dass das Rastelement an einer doppelseitigen Wippe ausgebildet ist, welche sich in Axialrichtung erstreckt und somit um eine Wipp-Achse schwenkbar ist, welche im Wesentlichen tangential bzw. senkrecht zu einer Mittelachse der chirurgischen Kupplungsvorrichtung angeordnet ist. Durch die Wippbewegung der Wippe kann somit das Rastelement mit der Verrastungsausnehmung in Eingriff gebracht bzw. von dieser freigegeben werden.

An dem anderen Ende, gegenüberliegend dem das Rastelement tragenden Ende, ist die Wippe mit einem Schwenkelement in Eingriff. Das Schwenkelement bestimmt somit die jeweilige Stellung der Wippe. Durch das Schwenkelement wird die Wippe somit in eine Verriegelungsposition bzw. eine Freigabeposition gebracht. In der Verriegelungsposition befindet sich das Rastelement in der Verrastungsausnehmung und verrastet somit das chirurgische Instrument mit dem Handstück. In der Freigabeposition befindet sich das Rastelement nicht in der Verrastungsausnehmung, so dass das chirurgische Instrument aus dem Handstück entfernt werden bzw. in dieses eingeschoben werden kann.

Durch eine gezielte Bewegung der Wippe erfolgt somit entweder eine Verrastung oder eine Freigabe.

Erfindungsgemäß ist vorgesehen, dass die Wippe mittels einem Schwenkelement betätigbar ist. Das Schwenkelement befindet sich hierzu, wie erwähnt, in Eingriff mit einem Ende der Wippe. Das Schwenkelement selbst ist in bevorzugter Ausgestaltung der Erfindung in eine Ruheposition vorgespannt, in welcher sich die Wippe in der Verriegelungsposition befindet. Durch eine Betätigung des Schwenkelements kann dieses in eine Freigabeposition verschwenkt werden. Dieses führt auch zu einer Verschwenkung der Wippe, da sich diese mit dem Schwenkelement in Eingriff befindet. Somit wird sich auch die Wippe in eine Freigabeposition bewegen.

In einer günstigen Ausgestaltung der Erfindung ist vorgesehen, dass das Schwenkelement mittels eines elastischen Elements in die Ruheposition vorgespannt ist. Das elastische Element kann in Form einer Feder ausgebildet sein, welche sich in einer Ausnehmung des Schwenkelements befindet. Zum Lösen des Rastelements aus der Verrastungsausnehmung ist es somit lediglich erforderlich, das Schwenkelement entsprechend zu verschwenken. Dies kann manuell auf einfache Weise erfolgen. Es sind somit keine komplizierten oder sensiblen Handhabungsmaßnahmen nötig, so dass die erfindungsgemäße chirurgische Kupplungsvorrichtung auch im operativen Umfeld leicht und einfach betätigt werden kann.

In besonders günstiger Ausgestaltung der Erfindung ist vorgesehen, dass die Wippe an ihrer einer Mittelachse der Kupplungsvorrichtung zugewandten Seite mit einem Lagerbereich versehen ist, welcher gegen eine Stützfläche des chirurgischen Instruments anliegt. Bevorzugterweise ist die Wippe angrenzend an den Lagerbereich beidseitig abgeschrägt. Der Lagerbereich bildet somit einen konvexen Bereich, der bevorzugterweise linienförmig ausgebildet ist. Hierdurch wird eine reibungsarme Bewegung der Wippe zwischen der Verriegelungsposition und der Freigabeposition gewährleistet.

Der Eingriffsbereich zwischen dem Schwenkelement und der Wippe ist bevorzugterweise so ausgebildet, dass die Wippe an ihrem dem Schwenkelement zugewandten Ende auskragend ausgebildet ist, so dass sich ein vorstehender Rand oder ein vorstehender zapfenförmiger Bereich ergibt. Dieser ist in einer Ausnehmung des Schwenkelements angeordnet, so dass sich ein Eingriff in der Art eines Gelenks ergibt. Hierdurch wird eine Schwenkbewegung des Schwenkelements auf die Wippe übertragen. Das Ende der Wippe wird hierzu in radialer Richtung, bezogen auf die Mittelachse der Kupplungsvorrichtung, bewegt.

Um eine Betätigung oder Handhabung des Schwenkelements zu erzielen, ist dieses bevorzugterweise an seiner der Mittelachse zugewandten Seite ebenfalls mit einem Lagerbereich versehen, der gegen eine Stützfläche des chirurgischen Instruments anliegt. Hierdurch kann das Schwenkelement verschwenkt oder gekippt werden. Dies führt zu der beschriebenen Bewegung der Wippe, um das Rastelement freizugeben bzw. zu verrasten.

Besonders günstig ist es, wenn das Schwenkelement mit einem Betätigungsbereich versehen ist, der in Form eines Knopfes oder eines Griffes ausgebildet ist. Dieser kann ausreichend über die Umfangsfläche des chirurgischen Instruments vorstehen, so dass das Schwenkelement jeweils sicher betätigbar ist.

Erfindungsgemäß ist somit eine chirurgische Kupplungsvorrichtung geschaffen, die es gestattet, das chirurgische Instrument sicher mit dem Handstück zu verrasten. Das Einschieben kann dabei entweder durch eine gezielte Betätigung der Wippe zum Freigeben des Rastelements oder nur durch Einschieben erfolgen. Im letzteren Falle wird das Rastelement, bedingt durch seine Abschrägung, selbsttätig in die Verrastungsausnehmung eingeführt. Zum Lösen ist es lediglich erforderlich, den Betätigungsbereich des Schwenkelements zu betätigen. Da die beiden Elemente (Wippe und Schwenkelement) getrennte Elemente bilden, ist zum einen eine sichere Funktion gewährleistet. Zum anderen ergibt sich eine einfache und kostengünstige Herstellbarkeit, da lediglich zwei Bauelemente zueinander zugeordnet werden müssen.

Im Folgenden wird die Erfindung anhand eines Ausführungsbeispiels in Verbindung mit der Zeichnung beschrieben. Dabei zeigt:

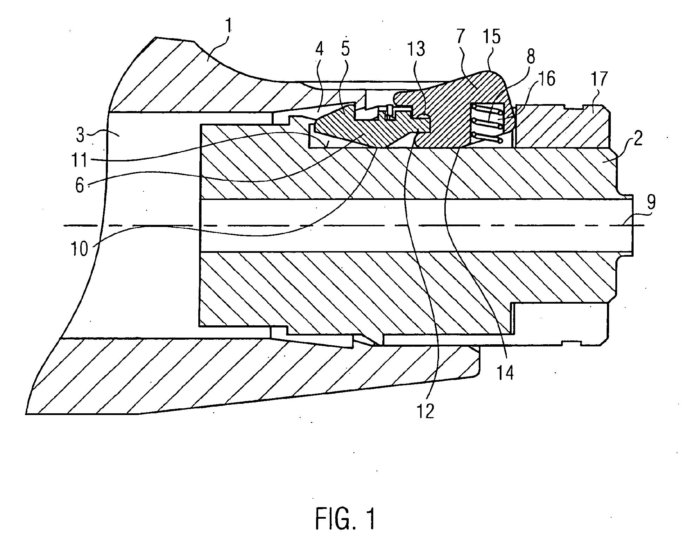

- Fig. 1: eine axiale Teil-Schnittansicht der erfindungsgemäße chirurgischen Kupplungsvorrichtung.

Die Fig. 1 zeigt einen zylindrischen Bereich eines Handstücks 1, welches mit einer im Wesentlichen zylindrischen zentrischen Ausnehmung 3 versehen ist. Eine Mittelachse des Handstücks 1 ist mit dem Bezugszeichen 9 dargestellt.

Das Handstück 1 kann beispielsweise eine Antriebseinrichtung oder ähnliches umfassen, die zur Koppelung und zum Antrieb eines chirurgischen Instruments 2 dienen kann.

Das chirurgische Instrument 2 ist in Fig. 1 nur schematisch im Teilbereich dargestellt. Es umfasst einen Endbereich, welcher im Wesentlichen zylindrisch ausgebildet ist und eine beispielsweise axial schlitzartige Ausnehmung mit einer Stützfläche 11 umfasst. In der im Einzelnen nicht dargestellten axial schlitzähnlichen Ausnehmung ist eine Wippe 6 angeordnet, die radial nach innen weisend einen erhöhten Lagerbereich 10 aufweist. Der Lagerbereich 10 kann eine Linienberührung mit der Stützfläche 11 hervorrufen. Es ist auch möglich, den Lagerbereich 10 mit einem Radius zu versehen, so wie dies in vereinfachter Darstellung in Fig. 1 gezeigt ist. Angrenzend an den Lagerbereich 10 ist die Wippe 6 jeweils abgeschrägt, um auf diese Weise eine Wipp-Bewegung ausführen zu können.

An ihrem linken Ende (bezogen auf Fig. 1) ist an der Wippe 6 ein hakenförmiges Rastelement 5 ausgebildet, welches in eine Verrastungsausnehmung 4 des Handstücks 1 einbringbar ist. Bei dem gezeigten Ausführungsbeispiel ist die Verrastungsausnehmung 4 in Form einer Ringnut ausgebildet. Es ist jedoch auch möglich, lediglich eine singuläre Ausnehmung vorzusehen.

Das - gemäß Fig. 1 - rechte Ende 12 der Wippe 6 ist in eine schlitzartige oder passend geformte Ausnehmung 13 eines Schwenkelements 7 lose eingesteckt. Es könnte jedoch auch eine scharnierartige axiale Fixierung vorgesehen werden.

Das Schwenkelement 7 ist an seiner radial nach innen weisenden Seite mit einem Lagerbereich 14 versehen, der ebenfalls eine Punktberührung oder Linienberührung oder Flachenberührung mit der Stützfläche 11 hat. Zumindest eine Seite des innenliegenden Bereichs des Schwenkelements 7 ist, wie in Fig. 1 dargestellt, abgeschrägt, um das Schwenkelement 7 entsprechend verschwenken zu können. Zur manuellen Betätigung umfasst das Schwenkelement 7 einen Betätigungsbereich 15. Weiterhin ist das Schwenkelement 7 mit einer Ausnehmung 16 versehen, in welcher sich ein elastisches Element (Feder) 8 befindet. Hierdurch wird das Schwenkelement 7 in eine Position vorgespannt, in welcher sich die Wippe 6 in der in Fig. 1 gezeigten Verriegelungsposition befindet. Durch ein Verschwenken oder Kippen des Schwenkelements 7 durch einen Diuck auf den Betätigungsbereich 15 erfolgt eine Verschwenkung oder Kippung, infolge derer die Ausnehmung 13 radial nach außen bewegt wird. Diese Bewegung nimmt das Ende 12 der Wippe 6 ebenfalls in radial nach außen gerichteter Richtung mit, wodurch die Wippe 6 um den Lagerbereich 10 verschwenkt wird. Dies wiederum führt dazu, dass das Rastelement 5 aus der Verrastungsausnehmung 4 gelöst wird.

Um die Wippe 6 und das Schwenkelement 7 in Position zu halten, ist auf dem chirurgischen Instrument 2 ein Sicherungselement 17 angeordnet, welches beispielsweise ringförmig oder teilzylindrisch ausgestaltet sein kann.

### Bezugszeichenliste

- 1: Handstück
- 2: Chirurgisches Instrument
- 3: Ausnehmung
- 4: Verrastungsausnehmung
- 5: Rastelement
- 6: Wippe
- 7: Schwenkelement
- 8: Elastisches Element/Feder
- 9: Mittelachse
- 10: Lagerbereich
- 11: Stützfläche
- 12: Ende
- 13: Ausnehmung
- 14: Lagerbereich
- 15: Betätigungsbereich
- 16: Ausnehmung
- 17: Sicherungselement

## Patentansprüche

1. Chirurgische Kupplungsvorrichtung zur lösbaren Verbindung eines Handstücks (1) mit einem chirurgischen Instrument (2), wobei das Handstück (1) mit einer Ausnehmung (3) versehen ist, in welche ein Kupplungsbereich des Instruments (2) lösbar einschiebbar ist, wobei an einer innenliegenden Wandung der Ausnehmung (3) eine Verrastungsausnehmung (4) ausgebildet ist, wobei die chirurgische kupplungsworrichtung ein Rastelement (5), eine doppelseitige Wippe (6) und ein Schwenkelement (7) umfasst, wobei das Rastelement (5) mit der Verrastungsausuchnung (4) lösbar in Eingriff bringbar und an dem Instrument (2) lagerbar ist, wobei das Rastelement (5) an der sich im Wesentlichen in Axialrichtung erstreckenden, doppelseitigen Wippe (6) ausgebildet ist, an deren einem Ende das Rastelement (5) angeordnet ist und deren anderes Ende (12) mit dem Schwenkelement (7) zur Betätigung der Wippe (6) in Eingriff ist.

2. Vorrichtung nach Anspruch 1, **dadurch gekennzeichnet, dass** das Schwenkelement (7) aus einer Ruheposition in eine Freigabeposition verschwenkbar ist.

3. Vorrichtung nach Anspruch 2, **dadurch gekennzeichnet, dass** das Schwenkelement (7) in die Ruheposition vorgespannt ist.

4. Vorrichtung nach Anspruch 2 oder 3, **dadurch gekennzeichnet, dass** das Schwenkelement (7) mittels eines elastischen Elements (8) in die Ruheposition vorgespannt ist.

5. Vorrichtung nach einem der Ansprüche 1 bis 4, **dadurch gekennzeichnet, dass** die Wippe (6) an ihrer einer Mittelachse (9) der Kupplungsvorrichtung zugewandten Seite mit einem Lagerbereich (10) versehen ist, welcher gegen eine Stützfläche (11) des chirurgischen Instruments anliegt.

6. Vorrichtung nach Anspruch 5, **dadurch gekennzeichnet, dass** die Wippe (6) angrenzend an den Lagerbereich (10) beidseitig abgeschrägt ist.

7. Vorrichtung nach einem der Ansprüche 1 bis 6, **dadurch gekennzeichnet, dass** das mit dem Schwenkelement (7) in Eingriff befindliche Ende (12) der Wippe (6) auskragend ausgebildet ist und in einer Ausnehmung (13) des Schwenkelements (7) angeordnet ist.

8. Vorrichtung nach einem der Ansprüche 1 bis 7, **dadurch gekennzeichnet, dass** das Schwenkelement (7) an seiner der Mittelachse (9) zugewandten Seite mit einem Lagerbereich (14) versehen ist, welcher gegen eine Stützfläche (11) des chirurgischen Instruments anliegt.

9. Vorrichtung nach Anspruch 8, **dadurch gekennzeichnet, dass** das Schwenkelement (7) angrenzend an den Lagerbereich (14) zumindest einseitig abgeschrägt ist.

10. Vorrichtung nach einem der Ansprüche 1 bis 9, **dadurch gekennzeichnet, dass** das Schwenkelement (7) mit einem Betätigungsbereich (15) versehen ist.

11. Vorrichtung nach einem der Ansprüche 4 bis 10, **dadurch gekennzeichnet, dass** das elastische Element (8) in Form einer Feder ausgebildet ist, welche in einer Ausnehmung (16) des Schwenkelements (7) angeordnet ist.

12. Vorrichtung nach einem der Ansprüche 1 bis 11, **dadurch gekennzeichnet, dass** das Rastelement (5) mit einer stirnseitigen Abschrägung versehen ist.

13. Chirurgisches Instrument mit einer Kupplungsvorrichtung nach einem der Ansprüche 1 bis 12.

## Claims

1. Surgical coupling device for the detachable connection of a handpiece (1) to a surgical instrument (2), wherein the handpiece (1) is provided with a recess (3), into which a coupling region of the instrument (2) can be detachably inserted, a locking recess (4) being configured on an inner wall of the recess (3), wherein the surgical coupling device comprises a locking element (5), a double-sided rocker (6) and a pivoting element (7), wherein the locking element (5) can be brought into engagement with the locking recess (4) in a detachable manner and can be mounted on the instrument (2), the locking element (5) being configured on the double-sided rocker (6) extending substantially in the axial direction, on the one end of which rocker the locking element (5) is arranged and the other end (12) of which is in engagement with the pivoting element (7) to actuate the rocker (6).

2. Device according to claim 1, **characterised in that** the pivoting element (7) can be pivoted from a rest position into a release position.

3. Device according to claim 2, **characterised in that** the pivoting element (7) is biased into the rest position.

4. Device according to claim 2 or 3, **characterised in that** the pivoting element (7) can be biased into the rest position by means of an elastic element (8).

5. Device according to any one of claims 1 to 4, **characterised in that** the rocker (6) is provided at its side facing a centre axis (9) of the coupling device with a bearing region (10), which rests against a support face (11) of the surgical instrument.

6. Device according to claim 5, **characterised in that** the rocker (6) is bevelled on either side adjacent to the bearing region (10).

7. Device according to any one of claims 1 to 6, **characterised in that** the end (12) of the rocker (6) which is in engagement with the pivoting element (7) is configured so as to project and is arranged in a recess (13) of the pivoting element (7).

8. Device according to any one of claims 1 to 7, **characterised in that** the pivoting element (7) is provided at its side facing the centre axis (9) with a bearing region (14), which rests against a support face (11) of the surgical instrument.

9. Device according to claim 8, **characterised in that** the pivoting element (7) adjacent to the bearing region (14) is bevelled at least on one side.

10. Device according to any one of claims 1 to 9, **characterised in that** the pivoting element (7) is provided with an actuating region (15).

11. Device according to any one of claims 4 to 10, **characterised in that** the elastic element (8) is configured in the form of a spring, which is arranged in a recess (16) of the pivoting element (7).

12. Device according to any one of claims 1 to 11, **characterised in that** the locking element (5) is provided with a bevel on the end face.

13. Surgical instrument with a coupling device according to any one of claims 1 to 12.

## Revendications

1. Dispositif d'accouplement chirurgical pour relier de manière séparable à un instrument chirurgical (2) une pièce à main (1), dans lequel :
- la pièce à main présente une cavité (3) dans laquelle peut être introduit par coulissement, de manière séparable, une zone d'accouplement de l'instrument chirurgical,
- sur une paroi interne de la cavité (3) se trouve un évidement de blocage (4),
- il y a un élément d'arrêt (5), une bascule (6) pouvant basculer des deux côtés et un élément de basculement (7),
- l'élément d'arrêt (5) peut être amené en prise, de manière séparable, avec la cavité de blocage (4) en étant maintenu sur l'instrument (5) et cet élément, qui s'étend essentiellement en direction axiale la bascule (6) présente vers une extrémité l'élément d'arrêt (5) tandis que l'autre extrémité (12) est en prise avec l'élément de basculement (7) qui sert à actionner cette bascule.

2. Dispositif selon la revendication 1, **caractérisé en ce que** l'élément de basculement (7) peut passer par basculement d'une position de repos à une position de libération.

3. Dispositif selon la revendication 2, **caractérisé en ce que** l'élément de basculement (7) est précontraint en direction de sa position de repos.

4. Dispositif selon la revendication 2 ou 3, **caractérisé en ce que** l'élément de basculement (7) est précontraint en direction de sa position de repos par un élément élastique (8).

5. Dispositif selon une des revendications 1 à 4, **caractérisé en ce que** sur son côté en regard de l'axe central (9) du dispositif d'accouplement, la bascule présente une zone de palier (10) qui est appliquée sur une portée de soutien (11) de l'instrument chirurgical.

6. Dispositif selon la revendication 5, **caractérisé en ce que** la bascule (6), en limite de la zone de palier (10) présente une pente sur chacun de ses deux côtés.

7. Dispositif selon une des revendications 1 à 6, **caractérisé en ce que** l'extrémité (12) de la bascule (6) qui est en prise avec l'élément de basculement (7) présente un collet qui est logé dans un évidement (B) de l'élément de basculement.

8. Dispositif selon une des revendications 1 à 7, **caractérisé en ce que** l'élément de basculement (7), sur son côté en regard de l'axe central (9) présente une zone de palier (14) qui est appliquée sur une portée de soutien (11) de l'instrument chirurgical.

9. Dispositif selon la revendication 8, **caractérisé en ce que** l'élément de basculement (7), en limite de la zone de palier (14) présente une pente sur au moins un de ses côtés.

10. Dispositif selon une des revendications 1 à 9, **caractérisé en ce que** l'élément de basculement (7) présente une zone d'actionnement (15).

11. Dispositif selon une des revendications 4 à 10, **caractérisé en ce que** l'élément élastique (8) est un ressort monté dans un évidement (16) de l'élément de basculement (7).

12. Dispositif selon une des revendications 1 à 11, **caractérisé en ce que** l'élément d'arrêt (5) présente, frontalement, une pente.

13. Instrument chirurgical équipé d'un dispositif d'accouplement selon une des revendications 1 à 12.
